Europäisches Patentamt

European Patent Office

Office européen des brevets

(11) Publication number: **0 336 201**

A1

## EUROPEAN PATENT APPLICATION

(21) Application number: 89105005.6

(22) Date of filing: 21.03.89

(51) Int. Cl.4: **A61B 5/00** , **A61B 5/08** , **G06F 15/42**

(30) Priority: 25.03.88 IT 1995788
11.11.88 IT 2260488

(43) Date of publication of application:
**11.10.89 Bulletin 89/41**

(84) Designated Contracting States:
**AT BE CH DE ES FR GB GR IT LI LU NL SE**

(71) Applicant: **BURKE & BURKE S.N.C. DI ANTONIO BURKE E JOHN BURKE**
**Via Paolo Boselli, 22/C**
**I-16146 Genova(IT)**

(72) Inventor: **Dal Negro, Roberto**
**Via Gabriele Rossetti, 4**
**I-37124 Verona(IT)**

(74) Representative: **Gervasi, Gemma, Dr.**
**Studio Brevetti e Marchi NOTARBARTOLO & GERVASI 33, Viale Bianca Maria**
**I-20122 Milano(IT)**

(54) Apparatus for conducting a diagnostic test.

(57) An apparatus for performing a diagnostic test is described which by measuring and processing a series of novel parameters enables early diagnosis to be made of bronchial asthma, bronchial hyper-reactivity even in asymptomatic subjects, and respiratory syndromes related thereto in any manner.

A suitable component (1) monitors the $TcPO_2$ and $TcPCO_2$ variations in the blood and the power required to maintain thermal stability of the electrode during all stages of a bronchial induction test. A suitable analog-digital converter (2) then feeds the signals to a suitably programmed processor (3 - 4) to provide real time data giving early warning of alterations in the bronchial structure.

FIG 2

## APPARATUS FOR CONDUCTING A DIAGNOSTIC TEST

This invention relates to an apparatus formed from several components which is useful diagnostically for determinations necessary for the study and early diagnosis of bronchial asthma, bronchial hyperreactivity and respiratory syndromes related thereto in any manner.

Specifically, the apparatus consists of a series of components connected together in a coordinated manner and arranged to measure the blood $O_2$ and $CO_2$ content and the power value (the power required for maintaining electrode thermal stability) and to transmit and then process the data obtained in order to provide not only qualitative but also quantitative information on the extent and development of alterations in the bronchial structures during the course of bronchial asthma or hyperreactivity to any stimulus, and is thus particularly suitable for the early indication of alterations not as yet clear.

The blood $O_2$ and $CO_2$ content are currently evaluated by analysing the artery blood or by a transdermic method.

The first method gives instantaneous values, ie corresponding to the moment in which the withdrawal from the artery is made, whereas the second method gives information on the variation in the concentration of the two gases with time.

The non-sanguinary transdermic method has become available relatively recently following the construction of special electrodes which are made to adhere to the skin, and because they are able to be heated (temperature in the range of 41-45 $^\circ$C) they arterialize the cutaneous venous blood (making it very similar to the actual artery blood) and enable $PO_2$ and $PCO_2$ concentrations to be measured which are comparable to those of the artery blood. Currently known systems are practically all similar in terms of their principle of operation and all comprise a controlled system for heating the electrode and measuring the energy required to keep the skin temperature chosen for the test constant.

The use transcutaneous monitoring of $TcPO_2$ and $TcPO_2$ (transcutaneous partial pressures) has been limited up to the present time mainly to two large fields of clinical application, namely the adult pneumological field for recognising the onset of hypoxemia and hypercapnia episodes, and the neonatal field for monitoring $O_2$ and $CO_2$ variations in the unborn child during labour or during the first hours of the baby's life.

The particular constituent parts of the apparatus according to the invention make it possible to continuously record the $TcPCO_2$, $TcPCO_2$ and power value, before, during and after any bronchial challenge or any pharmaceutical administration. By processing their combined patterns recorded during all stages of the bronchial hyperreactive test it is possible to evaluate parameters which allow precise diagnostic criteria to be obtained.

It has been observed that the time duration and quantifying of hypocapnia induced by bronchostimuli and the time duration and quantifying of the hypoxia which follows it a few minutes after the stimulus is suspended plus the power behaviour are parameters of fundamental predictive importance. The particular method of combined monitoring of hypocapnia, hypoxia and power requirement by the apparatus of the invention provides a highly discriminating method for the preventive evaluation of the onset of pulmonary function alterations which would otherwise not be identifiable using traditional functional (volume and flow parameters) and mechanical diagnostic methods. These are in fact unsuitable for sensing alterations of the bronchial structures which are not as yet clear or are in their initial stage, and are unsuitable for evaluating modifications dependent on non-muscular bronchial structures, ie dependent particularly on epithelium and vessels.

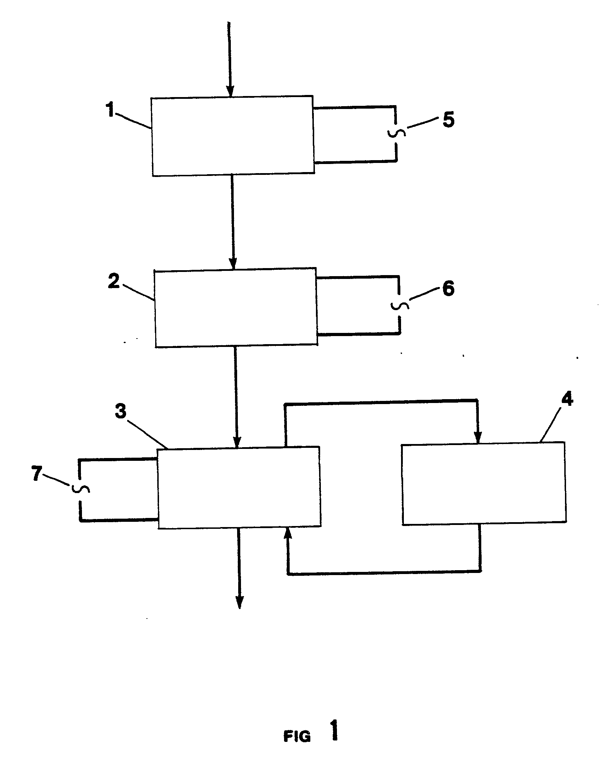

Figure 1 shows the block diagram of the apparatus according to the invention. Said Figure 1 and the details given hereinafter to illustrate the invention in no way represent a limitation thereon.

In Figure 1 a transdermic sensor 1 is shown for monitoring the $TcPO_2$ and $TcPCO_2$ contents and the power value for a patient affected by bronchial asthma or of an individual subjected to a hyperreactive bronchial test (physical exercise, aerosol, allergens, fog, cold air, vapour, dust, smoke, gas, cigarette smoke, and chemical, physical, pharmacological and professional agents). The reference numeral 2 represents an analog-digital converter and 3 represents an electronic processor with a mathematical algorithm 4. The electrical energy sources are indicated at 5, 6 and 7.

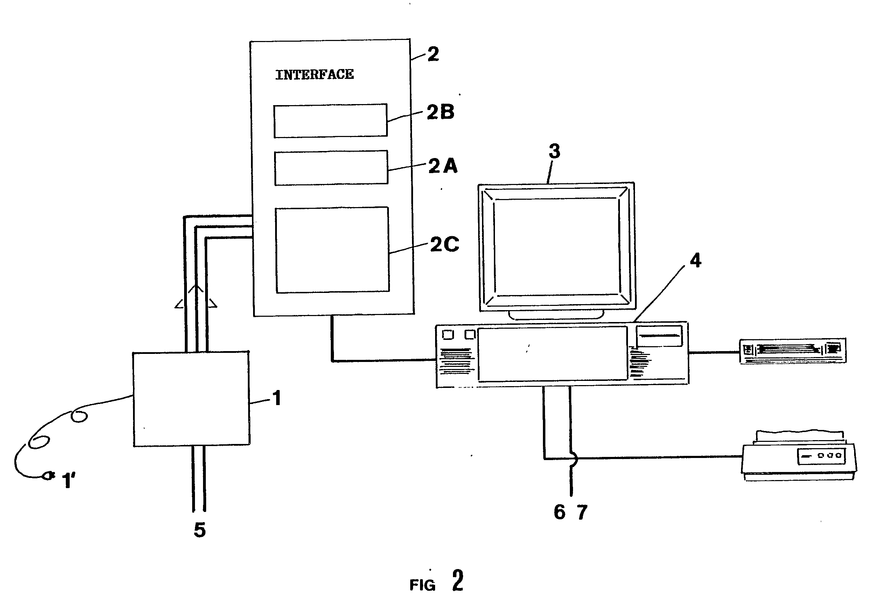

The apparatus according to the invention is shown in greater detail in Figure 2, in which operationally equivalent parts are identified by the same reference numerals as those used on the block diagram of Figure 1, the additional parts being as follows: 2A is the converter for converting an analog signal to a digital signal; 2B is the decoder for the digital signal to provide the weight in terms of bits; 2C is the sequencer which feeds the signals to the computer in logic sequence.

The computer 3 is an IBM-PC-AT compatible with 640 K of RAM, a 40 Mb (megabyte) hard disk, a 1.2 Mb floppy disk, and provided with an Ega graphic card, a 640x360 pixel graphic monitor and a colour graphic printer.

The reference numeral 4 indicates the data handling program which effects the following:
- it controls the converter in its acquisition, parameterization and graphic display of the data provided by the device 1
- it controls the patient clinical data (numerical and alpha-numerical) card
- it provides operating signals for the device 1
- it memorises in the data bank all the acquired values to allow subsequent mathematical-statistical processing
- it provides the final reports of the examinations carried out, in a form already parameterized and therefore directly readable.

During use, the electrode 1' of the transdermic sensor 1 is suitably connected to a low voltage electricity source 5 (battery or normal mains with transformer) and is applied to the skin of the patient suffering from bronchial asthma or of an individual to be subjected to a bronchial test after any bronchostimulus. The electrode is maintained at a temperature within the range of 41-45°C. On the basis of the $O_2$, $CO_2$ and power variations the transdermic sensor 1 emits electrical signals which are fed to the converter 2 where they are converted into digital signals and then into numerical signals. These latter are fed to the electronic processor 3 where they are read and evaluated on the basis of the system software 4.

Essentially, a series of three curves identified by different colours will appear on the processor screen in real time for the whole period of the test, which will generally have a maximum duration of 90 minutes per viewing.

The test will be considered to have commenced when the electrode has stabilized.

The curves are displayed as two types, one to an absolute scale of values and the other to a relative scale as a percentage of the basal pre-stimulus value.

The user can mount display and measurement templates on both scales.

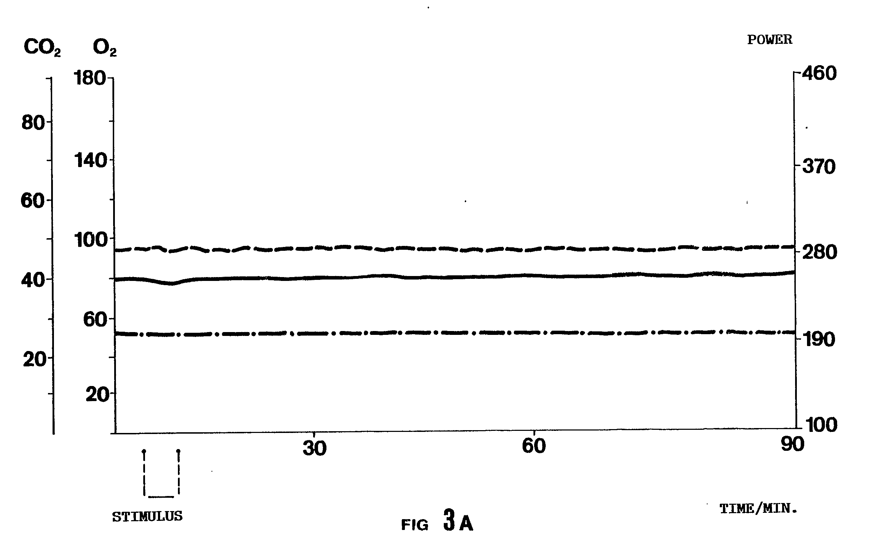

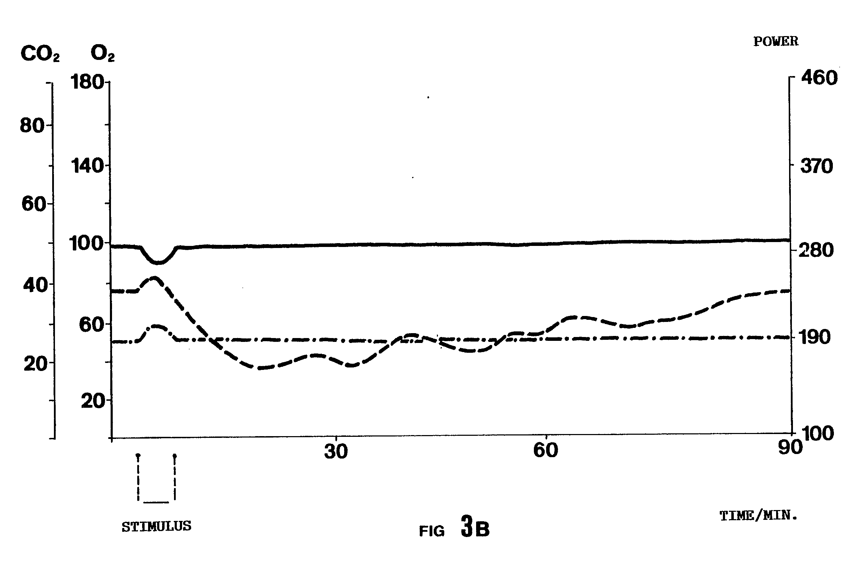

An absolute value curve is shown in Figures 3A and 3B. In these figures the dashed-line curve represents $TcPO_2$ variation, the full-line curve represents $TcPCO_2$ variation and the dotted curve represents power variation.

The curves of Figure 3A represent the analysis for a normal patient, whereas the curves of Figure 3B represent the analysis for an asymptomatic asthmatic patient.

It can be seen that for the normal subject the presence of the bronchostimulus does not induce significant variations in the three parameters, whereas for the asymptomatic asthmatic subject there is immediately after the stimulus an instantaneous decrease in the $PCO_2$ value and a simultaneous specular variation in power, both lasting for as long as the presence of the stimulus. The $PO_2$ parameter, after an initial transient increase, progressively decreases from its maximum, which occurs about 5-15 minutes after suspension of the stimulus, and without return to the basal value.

The extent and duration of the deflections in the three parameter curves indicates the extent of the pathological state of the bronchial structures.

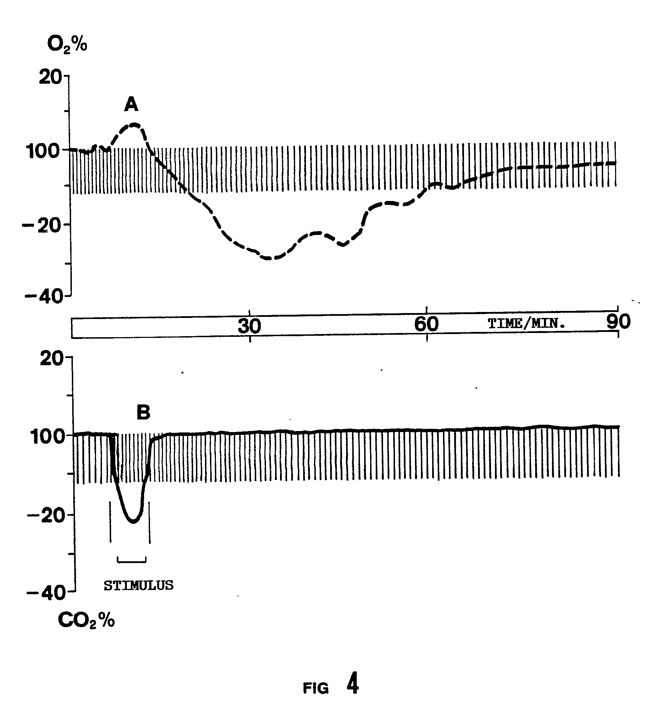

A relative percentage curve is shown in Figure 4. In this figure the $PO_2$ and $PCO_2$ values are shown as percentages of their basal pre-stimulus value, curve A being for oxygen and curve B for $CO_2$.

It can be seen that the shape of the curves is comparable to that of Figure 3B.

In the figure the area of statistical normality is shown by hatching.

The area of the $PO_2$ and $PCO_2$ curve below the area of statistical normality quantifies the extent of the reliably pathological response.

An evaluation of the curves and areas therefore allows identification of the response threshold for the individual subjects tested and enables normal responses to be distinguished from pathological responses. The fact that the measurements made are memorised at a high frequency per unit of time rather than on a polynomial basis means that the response of one and the same subject can be compared after therapeutic and prophylactic treatment. On the basis of a precise pharmacological model it is possible to identify the epithelium, muscular and bronchial vascularity alterations following the bronchostimulus, so overcoming the limits on the application and use of conventional transdermic $O_2$ and $CO_2$ sensors.

The parameters which the apparatus is able to monitor in real time are: time of onset of hypocapnia and hypoxia in response to the bronchostimulus; calculation of angular factors indicating the instantaneousness of the response; extent and duration of hypocapnia; extent and duration of hypoxia; extent of their maximum variation; time of their maximum quantitative expression; quantification of the time difference between the manifestation of the maximum variations for the two gases; quantification of the area subtended by the ideal line defined, by instantaneous comparison, on the basis of the pattern of the phenomena which characterise normal subjects; modifications in the patterns for the two gases following administration of particular test medicaments.

The apparatus therefore enables an early diagnosis of asthma and bronchial hyperreactive phenomena to be derived from an analysis of all these data, which up to the present time has not been possible.

The apparatus of the invention can be likewise applied to the study of animal models, and for monitoring also the O$_2$ and CO$_2$ gas exchange behaviour during drug administration in humans.

## Claims

1. An apparatus for effecting measurements which enable bronchial asthma, bronchial hyperreactivity for any stimulus and/or respiratory syndromes related thereto in any manner to be evaluated, characterised by comprising a transdermic sensor (1) for monitoring the TcPO$_2$ and TcPCO$_2$ contents of the patient's blood and the power required to maintain thermal stability of the electrode (1'), an analog-digital converter (2) connected to (1), and an electronic processor (3) connected to (2) and provided with a mathematical algorithm (4) able to provide the required information in real time.

2. An apparatus as claimed in claim 1, characterised in that said analog-digital converter (2) comprises the converter (2A) for converting an analog signal to a digital signal; the decoder (2B) for the digital signal to provide the weight in terms of bits, and the sequencer (2C) which feeds the signals to the computer in logic sequence.

3. An apparatus as claimed in claim 1, characterised in that said electronic processor (3) is an IBM-PC-AT compatible with 640 K of RAM, a 40 Mb hard disk, a 1.2 Mb floppy disk, and is provided with an Ega graphic card, a 640x360 pixel graphic monitor and a colour graphic printer.

4. An apparatus as claimed in claim 1, characterised in that said algorithm (4) controls the converter (2), controls the patient clinical data card, provides operating signals for the transdermic sensor (1), memorises into the data bank all the acquired values, and provides the final reports of the examinations carried out.

FIG 1

INTERFACE

2

2B

2A

2C

3

4

1

1'

5

6 7

FIG 2

EP 0 336 201 A1

FIG 3 A

STIMULUS

TIME/MIN.

CO₂  O₂

POWER

EP 0 336 201 A1

CO₂  O₂                                    POWER

STIMULUS                    FIG **3B**                    TIME/MIN.

EP 0 336 201 A1

FIG 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 171 927 (SUMITOMO ELECTRIC INDUSTRIES LTD.) * page 3, line 8 - page 5, line 37; page 7, lines 4 - 14; figure 1 * | 1 | A 61 B 5/00<br>A 61 B 5/08<br>G 06 F 15/42 |
| A | --- | 2,4 | |
| Y | JOURNAL OF PHYSICS E/SCIENTIFIC INSTRUMENTS) vol 29, no. 9, September 1987, pages 1103-1112, Bristol, GB; D. PARKER: "Sensors für monitoring blood gases in intensive care" * page 1106, right hand column, line 48 - page 1109, right hand column, line 6; figures 9,11,12 * | 1 | |
| Y | MEDICAL PROGRESS THROUGH TECHNOLOGY vol. 5, no. 4, 1978; pages 179-185; E. VOIGT: "Enlarged Acid-Base and Blood Gas Calculations by Electronical Data Computing in the Blood Gas Laboratory" * page 179, right hand column, lines 17-28; page 182, left hand column, line 15 - page 183, right hand column, line 53; figures 1,2,3 * | 1 | **TECHNICAL FIELDS SEARCHED (Int. Cl.4)**<br><br>A 61 B 5/00<br>G 06 F 15/00 |
| A | idem | 2,4 | |

The present search report has been drawn up for all claims

| Place of search | Date of completion of the search | Examiner |
|---|---|---|
| BERLIN | 19-06-1989 | WEIHS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                           
& : member of the same patent family, corresponding document

EPO FORM 1503 03.82 (P0401)